# EUROPEAN PATENT APPLICATION

(11) **EP 3 711 744 A1**
(43) Date of publication of application: **23.09.2020**
(21) Application number: 18877001.0
(22) Date of filing: 02.10.2018
(51) Int. Cl.: A61K 8/58, A61Q 1/06, A61Q 1/10, A61Q 17/04, A61Q 19/10

(54) **COSMETIC**

(30) Priority: 13.11.2017 JP 2017218440
(71) Applicant: Shin-Etsu Chemical Co., Ltd., Tokyo 100-0004 (JP)
(72) Inventor: MORIYA Hiroyuki, Annaka-shi Gunma 379-0224 (JP); KAMEI Masanao, Annaka-shi Gunma 379-0224 (JP)
(74) Representative: Wibbelmann, Jobst
(86) International application number: PCT/JP2018/036843
(87) International publication number: WO 2019/093019

(57) **Abstract**

The present invention is a cosmetic containing (A) an organo(poly)siloxane shown by the following general formula (1). This provides a cosmetic with a refreshing feeling, smooth spreadability as well as high stability and favorable cosmetic sustainability is provided. 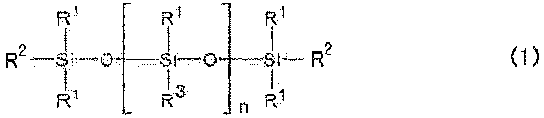 [where, in the formula, R¹ represents a group except shown by the formula (2), selected from a C1-C20 alkyl group and a C6-C20 aryl group; n represents an integer of 0 to 3; R² and R³ represent R¹ or the formula (2); and either or both of R² and R³ represent formula (2), 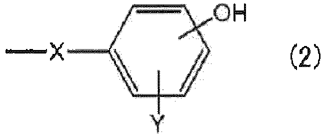 (where, in the formula, X represents a C2-C20 alkylene group; and Y represents any one of a hydrogen atom, a C1-C20 alkyl group, a C6-C20 aryl group, a C7-C40 aralkyl group, a C1-C20 alkyloxy group, a C6-C20 aryloxy group, a C7-C40 arylalkyloxy group, and a halogen atom.)]

## Description

### TECHNICAL FIELD

The present invention relates to a cosmetic.

### BACKGROUND ART

In sunscreen cosmetics, a UV-absorber is used, and they generally cause stickiness and oiliness on application. In particular, when using a solid organic UV-absorber, it is reported that stability is improved by the use of a highly polar oil or 4-tert-butyl-4'-methoxydibenzoylmethane, but there is oiliness, so there is a problem with feeling of use (Patent Documents 1 and 2) .

In contrast, among oil components, silicone oil has a refreshing feeling, and there have been attempts to use a silicone with an organic UV-absorber, but there are still problems regarding stability and feeling of use (Patent Documents 3 and 4).

In addition, a method in which a modified silicone is used has been suggested, but it cannot be considered sufficient (Patent Documents 5 and 6). Furthermore, there have been attempts to give a secondary-adhesion-less effect to cosmetics and attempts to improve the gloss of cosmetics by using a silicone containing a phenyl group, but it is disclosed that the silicone does not dissolve in an ester oil (Patent Document 7).

### CITATION LIST

### PATENT LITERATURE

Patent Document 1: Japanese Unexamined Patent Application Publication No. 2008-162988
Patent Document 2: Japanese Unexamined Patent Application Publication No. 2017-7969
Patent Document 3: Japanese Unexamined Patent Application Publication No. 2003-171253
Patent Document 4: Japanese Unexamined Patent Application Publication No. 2014-111583
Patent Document 5: Japanese Unexamined Patent Application Publication No. 2008-247897
Patent Document 6: Japanese Unexamined Patent Application Publication No. 2017-66140
Patent Document 7: Japanese Unexamined Patent Application Publication No. 2012-250927

### SUMMARY OF INVENTION

### TECHNICAL PROBLEM

The present invention has been made in view of the above situation, and an object of the present invention is to provide a cosmetic with a refreshing feeling, smooth spreadability as well as high stability and favorable cosmetic sustainability.

### SOLUTION TO PROBLEM

To solve the above problems, the present invention provides a cosmetic comprising: (A) an organo(poly)siloxane shown by the following general formula (1): 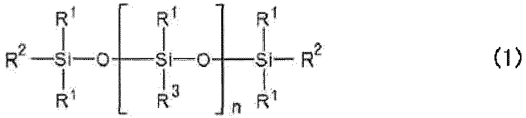 wherein, in the formula (1), R¹ independently represents a group except shown by the following general formula (2), selected from a group consisting of a substituted or unsubstituted alkyl group having 1 to 20 carbon atoms and a substituted or unsubstituted aryl group having 6 to 20 carbon atoms; n represents an integer of 0 to 3; R² and R³ each independently represent R¹ or an organic group shown by the following general formula (2), provided that either or both of R² and R³ in the formula (1) represent the organic group shown by the following general formula (2), 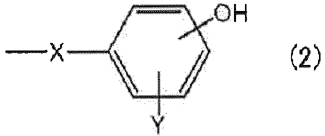 wherein, in the formula (2), X represents a substituted or unsubstituted alkylene group having 2 to 20 carbon atoms; Y represents any one selected from a group consisting of a hydrogen atom, a substituted or unsubstituted, linear, branched, or cyclic alkyl group having 1 to 20 carbon atoms, a substituted or unsubstituted aryl group having 6 to 20 carbon atoms, a substituted or unsubstituted aralkyl group having 7 to 40 carbon atoms, a substituted or unsubstituted alkyloxy group having 1 to 20 carbon atoms, a substituted or unsubstituted aryloxy group having 6 to 20 carbon atoms, a substituted or unsubstituted arylalkyloxy group having 7 to 40 carbon atoms, and a halogen atom; and an OH group in a phenyl group in the formula (2) substitutes independently at an ortho, meta, or para position.

Such a cosmetic provides a refreshing feeling, smooth spreadability as well as high stability and favorable cosmetic sustainability.

Furthermore, the inventive cosmetic preferably further comprises (B) an organic UV-absorber.

The inventive cosmetic does not cause stickiness or oiliness on application, and a refreshing feeling can be achieved, as well as high stability and favorable cosmetic sustainability, even when the organic UV-absorber (B) is contained.

In this case, (B) the organic UV-absorber is preferably one or more kinds selected from a group consisting of a triazine derivative, a benzoic ester derivative, a benzophenone derivative, and a dibenzoylmethane derivative.

Such an organic UV-absorber (B) has a high UV-protective effect, and is therefore preferable.

Furthermore, the inventive cosmetic preferably further comprises (C) a silicone oil other than (A) the organo(poly)siloxane.

Such a cosmetic containing the silicone oil (C) can achieve a further refreshing feeling as well as excellent stability.

Furthermore, a content of (A) the organo(poly)siloxane of the inventive cosmetic preferably is 0.1 to 50 mass%.

Such a cosmetic provides a refreshing feeling, smooth spreadability as well as high stability and favorable cosmetic sustainability with more certainty.

Furthermore, the inventive cosmetic preferably further comprises (D) an ester oil.

The organo(poly)siloxane (A) in the present invention dissolves in ester oil, and can therefore be used suitably in sunscreens and make-up cosmetics containing an ester oil.

### ADVANTAGEOUS EFFECTS OF INVENTION

The inventive cosmetic has a refreshing feeling peculiar to silicone oil and smooth spreadability while solving the problems of stability that conventional cosmetics have such as separation and precipitation, and thus having favorable cosmetic sustainability.

### DESCRIPTION OF EMBODIMENTS

To achieve the above object, the present inventors have earnestly studied and found out that a cosmetic (in particular, a sunscreen or a make-up cosmetic containing a UV-absorber or an ester oil) containing a particular silicone has a refreshing feeling peculiar to silicone oil, smooth spreadability as well as high stability and favorable cosmetic sustainability, and completed the present invention. Hereinafter, the inventive cosmetic will be described in detail.

That is, the present invention is a cosmetic containing: (A) an organo(poly)siloxane shown by the following general formula (1): 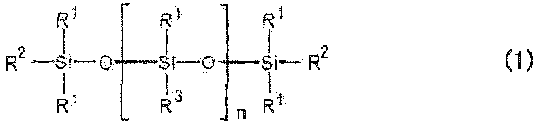 where, in the formula (1), R¹ independently represents a group except shown by the following general formula (2), selected from a group consisting of a substituted or unsubstituted alkyl group having 1 to 20 carbon atoms and a substituted or unsubstituted aryl group having 6 to 20 carbon atoms; n represents an integer of 0 to 3; R² and R³ each independently represent R¹ or an organic group shown by the following general formula (2), provided that either or both of R² and R³ in the formula (1) represent the organic group shown by the following general formula (2), 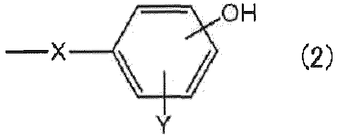 where, in the formula (2), X represents a substituted or unsubstituted alkylene group having 2 to 20 carbon atoms; Y represents any one selected from a group consisting of a hydrogen atom, a substituted or unsubstituted, linear, branched, or cyclic alkyl group having 1 to 20 carbon atoms, a substituted or unsubstituted aryl group having 6 to 20 carbon atoms, a substituted or unsubstituted aralkyl group having 7 to 40 carbon atoms, a substituted or unsubstituted alkyloxy group having 1 to 20 carbon atoms, a substituted or unsubstituted aryloxy group having 6 to 20 carbon atoms, a substituted or unsubstituted arylalkyloxy group having 7 to 40 carbon atoms, and a halogen atom; and an OH group in a phenyl group in the formula (2) substitutes independently at an ortho, meta, or para position.

Note that in the present invention, organo(poly)siloxane refers to organosiloxane which has one siloxane bond (-Si-O-Si-) per molecule and organopolysiloxane which has two or more siloxane bonds per molecule.

In the organo(poly)siloxane of the formula (1), R¹ independently represents a group except shown by the general formula (2), selected from a group consisting of a substituted or unsubstituted alkyl group having 1 to 20 carbon atoms and a substituted or unsubstituted aryl group having 6 to 20 carbon atoms. Examples of the unsubstituted alkyl group include a methyl group, an ethyl group, and a propyl group. Examples of the substituted alkyl group include a halogenated alkyl group. This halogenated alkyl group may be monohalogenated, or polyhalogenated, and may be a perhalogenated alkyl group. The halogen is preferably fluorine or chlorine, and fluorine is particularly preferable. Examples of substituents other than a halogen include aryl groups such as phenyl and tolyl. Examples of the substituted or unsubstituted aryl group having 6 to 20 carbon atoms include unsubstituted aryl groups such as a phenyl group and a naphthyl group and aryl groups in which hydrogen atoms thereof are substituted by a halogen or the like. A preferable R¹ includes a methyl, phenyl, and naphthyl group.

R² and R³ each independently represent R¹ or an organic group shown by the general formula (2). Note that either or both of R² and R³ in the formula (1) represent the organic group shown by the general formula (2). "n" represents an integer of 0 to 3, and preferably 0 to 2.

X independently represents a substituted or unsubstituted alkylene group having 2 to 20 carbon atoms, and may be any of linear, branched, or cyclic. X preferably represents a substituted or unsubstituted alkylene group having 2 to 12 carbon atoms, particularly preferably, a substituted or unsubstituted alkylene group having 3 to 8 carbon atoms. When the alkylene group has a substituent, examples of the substituent include halogen atoms such as fluorine and chlorine, and aryl groups such as phenyl and tolyl. Groups particularly preferable as X include propylene, isopropylene, butylene, and isobutylene groups.

Y independently represents any one selected from a group consisting of a hydrogen atom, a substituted or unsubstituted, linear, branched, or cyclic alkyl group having 1 to 20 carbon atoms, a substituted or unsubstituted aryl group having 6 to 20 carbon atoms, a substituted or unsubstituted aralkyl group having 7 to 40 carbon atoms, for example, an aralkyl group in which a hydrogen atom in an alkyl group having 1 to 20 carbon atoms is substituted by an aryl group having 6 to 20 carbon atoms (that is, a C6-C20 aryl C1-C20 alkyl), a substituted or unsubstituted alkyloxy group having 1 to 20 carbon atoms, a substituted or unsubstituted aryloxy group having 6 to 20 carbon atoms, a substituted or unsubstituted arylalkyloxy group having 7 to 40 carbon atoms, for example, an arylalkyloxy group in which a hydrogen atom in an alkyloxy group having 1 to 20 carbon atoms is substituted by an aryl group having 6 to 20 carbon atoms (that is, a C6-C20 aryl C1-C20 alkyloxy), and a halogen atom. Substituents thereof include those described above as substituents of the alkylene group X, for example, a halogen group such as fluorine. Furthermore, as Y, a hydrogen atom and a methoxy group are preferable. In addition, the OH group in the phenyl group in the formula (2) substitutes independently at an ortho, meta, or para position relative to X, particularly preferably at an ortho position or a para position.

Furthermore, as such an organo(poly)siloxane (A), an organo(poly)siloxane shown by the following general formulae (3) and (4) is preferable. 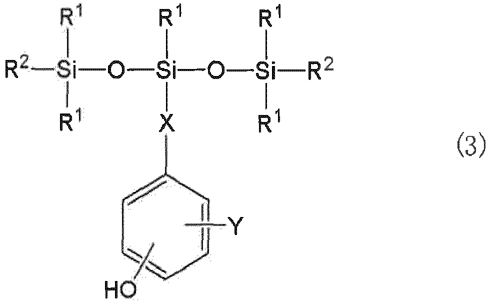 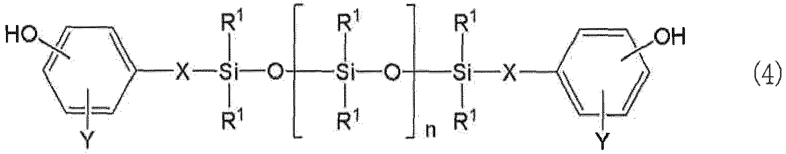 In the formula, R¹, R², X, Y, and n are the same as above.

The organo(poly)siloxane (A) shown by the general formula (1) may be used alone or in combination of two or more kinds.

In addition, the organo(poly)siloxane (A) used in the present invention can be synthesized with reference to methods disclosed in JP H6-184310 and WO 2014/073605.

Furthermore, the organo(poly)siloxane (A) in the inventive cosmetic may have a content of 0.1 to 50 mass% of the total cosmetic, preferably 1 to 30 mass%, more preferably 1 to 20 mass%.

The inventive cosmetic preferably further contains (B) an organic UV-absorber. The organic UV-absorber (B) is a UV-absorber having the function of protecting the skin from ultraviolet rays. As an organic UV-absorber that can be used in the present invention, at least one kind selected from a group consisting of triazine derivatives, benzoic ester derivatives, benzophenone derivatives, and dibenzoylmethane derivatives is preferable.

Examples of the triazine derivatives include 2,4-bis-{[4-(2-ethyl-hexyloxy)-2-hydroxy]-phenyl}-6-(4-methoxyphenyl)-1,3,5- triazine (Tinosorb S manufactured by BASF), 2,4,6-tris[4-(2-ethylhexyloxycarbonyl)anilino]-1,3,5-triazine (Uvinul T-150 manufactured by BASF or Helio sunOTZ manufactured by O'Laughlin Industries Co., Ltd.), and diethylhexylbutamidotriazine (Uvasorb HEB manufactured by 3V SIGMA). 2,4-bis-{[4-(2-ethyl-hexyloxy)-2-hydroxy]-phenyl}-6-(4-methoxyphenyl)-1,3,5-triazine and 2,4,6-tris[4-(2-ethylhexyloxycarbonyl)anilino]-1,3,5-triazine are preferable.

Examples of the benzoic ester derivatives include 2-[4-(diethylamino)-2-hydroxybenzoyl]benzoic acid hexyl ester (Uvinul A plus Granular, Uvinul A plus B manufactured by BASF).

Examples of the benzophenone derivatives include 2-hydroxy-4-methoxybenzophenone (Uvinul M40 manufactured by BASF, Escalol 567 manufactured by ISP), dihydroxy dimethoxybenzophenone (SEESORB 107 manufactured by Shipro Kasei Kaisha, Ltd.), dihydroxybenzophenone (SEESORB 100 manufactured by Shipro Kasei Kaisha, Ltd.), and tetrahydroxybenzophenone (SEESORB 106 manufactured by Shipro Kasei Kaisha, Ltd.).

Examples of the dibenzoylmethane derivatives include 4-tert-butyl-4'-methoxy-dibenzoylmethane (Parsol 1789 manufactured by DSM Nutrition Japan).

As the organic UV-absorber (B) in the present invention, at least one kind selected from a group consisting of 2,4-bis-{[4-(2-ethyl-hexyloxy)-2-hydroxy]-phenyl}-6-(4-methoxyphenyl)-1,3,5-triazine, 2,4,6-tris[4-(2-ethylhexyloxycarbonyl)anilino]-1,3,5-triazine, 2-[4-(diethylamino)-2-hydroxybenzoyl]benzoic acid hexyl ester, and 4-tert-butyl-4'-methoxy-dibenzoylmethane is preferable from the viewpoint of UV-protective effect or versatility as a raw material.

As the organic UV-absorber (B) in the present invention, a cinnamate-based UV-absorber, for example, octyl methoxycinnamate (ethylhexyl paramethoxycinnamate), ethyl-4-isopropylcinnamate, methyl-2,5-diisopropylcinnamate, ethyl-2,4-diisopropylcinnamate, methyl-2,4-diisopropylcinnamate, propyl-p-methoxycinnamate, isopropyl-p-methoxycinnamate, isoamyl-p-methoxycinnamate, octyl-p-methoxycinnamate (2-ethylhexyl-p-methoxycinnamate), 2-ethoxyethyl-p-methoxycinnamate, cyclohexyl-p-methoxycinnamate, ethyl-α-cyano-β-phenylcinnamate, 2-ethylhexyl-α-cyano-β-phenylcinnamate, and glycerylmono-2-ethylhexanoyl-diparamethoxycinnamate, may be further contained.

A content of the organic UV-absorber (B) in the present invention may be 0.1 to 50 mass% of the total cosmetic, preferably 0.5 to 30 mass%, further preferably 1 to 20 mass%.

Furthermore, the inventive cosmetic preferably contains (C) a silicone oil other than the organo(poly)siloxane (A). Examples of the silicone oil include volatile silicone oil. Examples of the volatile silicone oil include dimethylpolysiloxane (dimer, trimer, tetramer, pentamer), caprylyl methicone, octamethyl cyclotetrasiloxane, decamethyl cyclopentasiloxane, dodecamethylcyclohexasiloxane, tetramethyl tetrahydrogen cyclotetrasiloxane, tristrimethyl siloxymethylsilane, and tetrakis trimethylsiloxysilane; preferably, dimethylpolysiloxane (tetramer, pentamer), caprylyl methicone, decamethyl cyclopentasiloxane, dodecamethyl cyclohexasiloxane, tristrimethyl siloxymethylsilane, and tetrakis trimethylsiloxysilane. Furthermore, examples include low viscous to high viscous linear or branched organopolysiloxanes such as nonvolatile dimethylpolysiloxane, phenyl trimethicone, methyl phenyl polysiloxane, methylhexyl polysiloxane, methyl hydrogen polysiloxane, and dimethylsiloxane/methylphenylsiloxane copolymer; silicone rubber such as tetramethyltetraphenyl cyclotetrasiloxane, amino-modified organopolysiloxane, gummy dimethylpolysiloxane with a high degree of polymerization, gummy amino-modified organopolysiloxane, and gummy dimethylsiloxane/methylphenylsiloxane copolymer; and cyclic siloxane solution of silicone gum and rubber, trimethylsiloxysilicate, cyclic organopolysiloxane solution of trimethylsiloxysilicate, higher alkoxy-modified organopolysiloxane such as stearoxysilicone, higher fatty acid-modified organopolysiloxane, alkyl-modified organopolysiloxane, long chain alkyl-modified organopolysiloxane, fluorine-modified organopolysiloxane, silicone resin and a dissolution of a silicone resin; preferably, low viscous to high viscous linear or branched organopolysiloxanes such as nonvolatile dimethyl polysiloxane, phenyl trimethicone, methyl phenyl polysiloxane, methylhexyl polysiloxane, methyl hydrogen polysiloxane, and dimethylsiloxane/methylphenylsiloxane copolymer. A content of these silicone oils (C) may be 1 to 70 mass% of the total cosmetic, preferably 1 to 50 mass%, further preferably 5 to 30 mass%.

The inventive cosmetic preferably further contains (D) an ester oil. Examples of the ester oil (D) include diisobutyl adipate, di-2-hexyldecyl adipate, di-2-heptylundecyl adipate, N-alkylglycol monoisostearate, isocetyl isostearate, trimethylolpropane triisostearate, ethylene glycol di-2-ethylhexanoate, cetyl 2-ethylhexanoate, trimethylolpropane tri-2-ethylhexanoate, pentaerythritol tetra-2-ethylhexanoate, cetyl octanoate, octyl dodecyl gum ester, oleyl oleate, octyldodecyl oleate, decyl oleate, neopentyl glycol dioctanoate, neopentyl glycol dicaprate, triethyl citrate, di-2-ethylhexyl succinate, isocetyl stearate, butyl stearate, diisopropyl sebacate, di-2-ethylhexyl sebacate, cetyl lactate, myristyl lactate, isononyl isononanoate, isotridecyl isononanoate, isopropyl palmitate, 2-ethylhexyl palmitate, 2-hexyldecyl palmitate, 2-heptylundecyl palmitate, cholesteryl 12-hydroxystearate, a dipentaerythritol fatty acid ester, isopropyl myristate, octyldodecyl myristate, 2-hexyldecyl myristate, myristyl myristate, hexyldecyl dimethyloctanoate, ethyl laurate, hexyl laurate, di-2-octyldodecyl N-lauroyl-L-glutamate, isopropyl lauroyl sarcosinate, diisostearyl malate, acetoglyceryl, glyceryl triisooctanoate, glyceryl triisostearate, glyceryl triisopalmitate, glyceryl monostearate, glyceryl di-2-heptylundecanoate, glyceryl trimyristate, diglyceryl myristate isostearate, and butyl acetate. Furthermore, examples include an avocado oil, a linseed oil, an almond oil, an insects wax, a perilla oil, an olive oil, a cacao butter, a kapok wax, a kaya oil, a carnauba wax, a liver oil, a candelilla wax, a purified candelilla wax, a beef tallow, a neat's-foot oil, a beef bone fat, a cured beef tallow, an apricot kernel oil, a whale wax, a hydrogenated oil, a wheat germ oil, a sesame oil, a rice germ oil, a rice bran oil, a sugarcane wax, a sasanqua oil, a safflower oil, a shea butter, a Chinese tung oil, a cinnamon oil, a jojoba wax, a squalane oil, a squalene oil, a shellac wax, a turtle oil, a soybean oil, a tea seed oil, a camellia oil, an evening primrose oil, a corn oil, a pig fat, a rapeseed oil, a Japanese tung oil, a bran wax, a germ oil, a horse wax, a Persic oil, a palm oil, a palm kernel oil, a castor oil, a cured castor oil, a methyl ester of cured castor oil fatty acid, a sunflower oil, a grape seed oil, a bayberry wax, a jojoba oil, hydrogenated jojoba oil, a macadamia nut oil, a bees wax, a mink oil, a meadow foam oil, a cotton seed oil, a cotton wax, a Japan wax, a Japan wax kernel oil, a montan wax, a coconut oil, a cured coconut oil, a tri-coconut oil fatty acid glyceride, a mutton tallow, a peanut oil, lanolin, liquid lanolin, reduced lanolin, hard lanolin, lanolin acetate, lanolin alcohol acetate, and isopropyl lanolin fatty acid. A content of these ester oils (D) may be 0.5 to 50 mass% of the total cosmetic, preferably 1 to 30 mass%, further preferably 2 to 20 mass%.

Further, an oil material that is allowable for cosmetics can be contained in the inventive cosmetic. Other than those described above, examples of such a liquid oil material include hydrocarbon oil, higher fatty acid, higher alcohol, and fluorinated oil. Examples of the hydrocarbon oil include α-olefin oligomer, light isoparaffin, isododecane, light liquid isoparaffin, nonvolatile ozocerite, squalane, synthetic squalane, vegetable squalane, squalene, ceresin, paraffin, paraffin wax, polyethylene wax, polyethylene/polypropylene wax, (ethylene/propylene/styrene) copolymer, (butylene/propylene/styrene) copolymer, liquid paraffin, liquid isoparaffin, pristane, polyisobutylene, hydrogenated polyisobutene, microcrystalline wax, and vaseline. Examples of the higher fatty acid include lauric acid, myristic acid, palmitic acid, stearic acid, behenic acid, undecylenic acid, oleic acid, linoleic acid, linolenic acid, arachidonic acid, eicosapentaenoic acid (EPA), docosahexaenoic acid (DHA), isostearic acid, and 12-hydroxystearic acid. Examples of the higher alcohol include lauryl alcohol, myristyl alcohol, palmityl alcohol, stearyl alcohol, behenyl alcohol, hexadecyl alcohol, oleyl alcohol, isostearyl alcohol, hexyl dodecanol, octyl dodecanol, cetostearyl alcohol, 2-decyl tetradecynol, cholesterol, phytosterol, POE cholesterol ether, monostearyl glycerin ether (batyl alcohol), and monooleyl glyceryl ether (selachyl alcohol). Examples of the fluorinated oil material include perfluoro polyether, perfluoro decalin, and perfluoro octane. The amount of oil material allowable for cosmetics varies depending on the form of the cosmetic, but may be 1 to 98 mass% of the total cosmetic, and the range of 1 to 50 mass% is favorable.

The inventive cosmetic may have water blended in accordance with the object.

The inventive cosmetic may further contain one or two or more kinds of surfactant. The inventive cosmetic becomes a cosmetic excellent in usability by blending surfactant in accordance with the object. The surfactant includes anionic, cationic, nonionic, and amphoteric surfactant, but the surfactant contained in the inventive cosmetic is not particularly limited, and any surfactant that is used in ordinary cosmetics can be used.

Specific examples of the anionic surfactant include fatty acid soap such as sodium stearate and triethanolamine palmitate, alkyl ether carboxylic acid and a salt thereof, a condensation salt of amino acid and fatty acid, an alkane sulfonate salt, an alkene sulfonate, a sulfonate salt of fatty acid ester, a sulfonate salt of fatty acid amide, a sulfonate salt of formalin condensate, an alkyl sulfate ester salt, a sulfate ester salt of secondary higher alcohol, a sulfate ester salt of alkyl and allyl ether, a sulfate ester salt of fatty acid ester, a sulfate ester salt of fatty acid alkylolamide, sulfate ester salts of Turkey red oil or the like, an alkyl phosphate salt, an ether phosphate salt, an alkyl allyl ether phosphate salt, an amide phosphate salt, an N-acyl lactate salt, an N-acylsarcosinate salt, and an N-acylamino acid activator. Specific examples of the cationic surfactant include an alkylamine salt, an amine salt such as a polyamine or aminoalcohol fatty acid derivative, an alkyl quaternary ammonium salt, an aromatic quaternary ammonium salt, a pyridium salt, and an imidazolium salt.

Examples of the nonionic surfactant include sorbitan fatty acid ester, glycerin fatty acid ester, polyglycerin fatty acid ester, propylene glycol fatty acid ester, polyethylene glycol fatty acid ester, sucrose fatty acid ester, methyl glucoside fatty acid ester, alkyl polyglucoside, polyoxyethylene alkyl ether, polyoxypropylene alkyl ether, polyoxyethylene alkyl phenyl ether, polyoxyethylene fatty acid ester, polyoxyethylene sorbitan fatty acid ester, polyoxyethyelene sorbitol fatty acid ester, polyoxyethylene glycerin fatty acid ester, polyoxyethylene propylene glycol fatty acid ester, polyoxyethylene castor oil, polyoxyethylene hard castor oil, polyoxyethylene phytostanol ether, polyoxyethylene phytosterol ether, polyoxyethylene cholestanol ether, polyoxyethylene cholesterol ether, linear or branched polyoxyalkylene-modified organopolysiloxane, linear or branched organopolysiloxane co-modified with polyoxyalkylene and alkyl, linear or branched polyglycerin-modified organopolysiloxane, linear or branched organopolysiloxane co-modified with polyglycerin and alkyl, alkanol amide, sugar ether, and sugar amide.

Examples of the amphoteric surfactant include betaine, an aminocarboxylic acid salt, an imidazoline derivative, and an amide amine type.

Among these surfactants, preferable surfactants include linear or branched organopolysiloxane having a polyoxyethylene chain in the molecule, linear or branched organopolysiloxane having a polyglycerin chain in the molecule, and surfactants of these organopolysiloxane co-modified with alkyl. The commercial products thereof include KF-6011, KF-6011P, KF-6043, KF-6012, KF-6013, KF-6015, KF-6016, KF-6017, KF-6028, KF-6028P, KF-6038, KF-6100, KF-6104, and KF-6105 (all of which are manufactured by Shin-Etsu Chemical Co., Ltd.), but are not limited thereto. In addition, the surfactant preferably has HLB of 2 to 10, and the amount to be blended is preferably 0.1 to 20 mass% of the total cosmetic, and the range of 0.2 to 10 mass% is particularly favorable.

The inventive cosmetic may further contain one or two or more kinds of powder. Any powder may be used if it is usable for ordinary cosmetics regardless of its form (spherical, needle-like, plate-like, etc.), its particle diameter (fumed, microparticle, pigment-class, etc.), and its particle structure (porous, non-porous, etc.). Examples thereof include inorganic powder, organic powder, surfactant metal salt powder, and coloring agents such as color pigment, pearl pigment, tar dye, metal powder pigment, natural pigment, and dyes.

Specific examples of the inorganic powder include titanium oxide, zirconium oxide, zinc oxide, cerium oxide, magnesium oxide, barium sulfate, calcium sulfate, magnesium sulfate, calcium carbonate, magnesium carbonate, talc, mica, kaolin, sericite, muscovite, synthetic mica, phlogopite, lepidolite, biotite, lithia mica, silicic acid, anhydrous silicic acid, aluminum silicate, magnesium silicate, aluminum magnesium silicate, calcium silicate, barium silicate, strontium silicate, a metal tungstate salt, hydroxy apatite, vermiculite, higilite, bentonite, montmorillonite, hectorite, zeolite, ceramics powder, dibasic calcium phosphate, alumina, aluminum hydroxide, boron nitride, silica, and silica silylate.

Examples of the organic powder include polyamide powder, polyacrylic acid/acrylic ester powder, polyester powder, polyethylene powder, polypropylene powder, polystyrene powder, polyurethane powder, benzoguanamine powder, polymethyl benzoguanamine powder, tetrafluoroethylene powder, polymethyl methacrylate powder, cellulose powder, silk powder, nylon powder (powders such as 12 nylon and 6 nylon), crosslinked spherical dimethylpolysiloxane microparticles having a structure of crosslinked dimethylpolysiloxane, crosslinked spherical polymethylsilsesquioxane microparticles, crosslinked spherical organopolysiloxane rubber microparticles with the surface being coated with polymethylsilsesquioxane particles, hydrophobic silica, and a powder selected from styrene/acrylic acid copolymer, divinyl benzene/styrene copolymer, a vinyl resin, an urea resin, a phenolic resin, a fluororesin, a silicone resin, an acrylic resin, a melamine resin, an epoxy resin, and a polycarbonate resin; fine crystalline fiber powder, starch powder, powder of starch derivative of fatty acid, and lauroyl lysine.

Examples of the surfactant metal salt powder (metal soap) include zinc undecylate, aluminum isostearate, zinc stearate, aluminum stearate, calcium stearate, magnesium stearate, zinc myristate, magnesium myristate, zinc cetylphosphate, calcium cetylphosphate, sodium zinc cetylphosphate, zinc palmitate, aluminum palmitate, and zinc laurate.

Specific examples of the color pigment include inorganic red pigment such as iron oxide, iron hydroxide, and iron titanate; an inorganic brown pigment such as γ-iron oxide; inorganic yellow pigment such as yellow iron oxide and yellow earth; inorganic black pigment such as black iron oxide and carbon black; inorganic purple pigment such as manganese violet and cobalt violet; inorganic green pigment such as chromium hydroxide, chromium oxide, cobalt oxide, and cobalt titanate; inorganic blue pigment such as Prussian blue and ultramarine blue; a laked tar dye; a laked natural dye; and synthetic resin powder obtained by hybridization of these powders.

Specific examples of the pearl pigment include muscovite coated with titanium oxide, mica coated with titanium oxide, oxychloro bismuth, oxychloro bismuth coated with titanium oxide, a talc coated with titanium oxide, a fish scale foil, and color mica coated with titanium oxide. Examples of the metal powder pigment include aluminum powder, copper powder, and stainless powder.

Examples of the tar dye include Red No. 3, Red No. 104, Red No. 106, Red No. 201, Red No. 202, Red No. 204, Red No. 205, Red No. 220, Red No. 226, Red No. 227, Red No. 228, Red No. 230, Red No. 401, Red No. 505, Yellow No. 4, Yellow No. 5, Yellow No. 202, Yellow No. 203, Yellow No. 204, Yellow No. 401, Blue No. 1, Blue No. 2, Blue No. 201, Blue No. 404, Green No. 3, Green No. 201, Green No. 204, Green No. 205, Orange No. 201, Orange No. 203, Orange No. 204, Orange No. 206, and Orange No. 207. Examples of the natural dye include powder selected from carminic acid, laccaic acid, carthamin, brazilin, and crocin.

Among these powders, preferable ones for the present invention include crosslinked spherical dimethylpolysiloxane microparticles having a structure of at least partly crosslinked dimethylpolysiloxane, crosslinked spherical polymethylsilsesquioxane microparticles, crosslinked spherical polysiloxane rubber microparticles with the surface being coated with polymethylsilsesquioxane particles, crosslinked spherical diphenylpolysiloxane rubber microparticles with the surface being coated with polymethylsilsesquioxane particles, and hydrophobic silica; in addition, powder and coloring agents containing a fluorinated group are also usable. The commercial products thereof include KMP-590, KSP-100, KSP-101, KSP-102, KSP-105, and KSP-300 (all of which are manufactured by Shin-Etsu Chemical Co., Ltd.).

These powders are usable as a composite thereof or after being treated with general oil material, silicone oil, a fluorine compound, and/or surfactant. For example, they may or may not be previously subjected to surface treatment such as treatment with a fluorine compound, treatment with a silicone resin, treatment for pendant, treatment with a silane coupling agent, treatment with a titanate coupling agent, treatment with oil material, treatment with N-acylated lysine, treatment with polyacrylic acid, treatment with metal soap, treatment with amino acid, treatment with an inorganic compound, plasma treatment, and mechanochemical treatment. In accordance with needs, one or two or more kinds of them may be used. The amount of the powder to be added is preferably in the range of 99 mass% or less of the total cosmetic. In particular, the amount to be added is preferably in the range of 80 to 99 mass% of the total cosmetic in the case of powder cosmetic.

In the present invention, it is possible to further contain one or two or more kinds of (E) compound(s) each having an alcoholic hydroxy group in the molecular structure. Examples of such a compound include lower alcohol such as ethanol and isopropanol; sugar alcohol such as sorbitol and maltose; sterol such as cholesterol, sitosterol, phytosterol, and lanosterol; and polyhydric alcohol such as butylene glycol, propylene glycol, dibutylene glycol, and pentylene glycol, in which water soluble monohydric alcohol and water soluble polyhydric alcohol are generally used in many cases. The cosmetic favorably contains the compound having an alcoholic hydroxy group in the molecular structure in an amount of 98 mass% or less of the total cosmetic.

The present invention may further contain a composition composed of liquid oil material and one or two or more kinds of crosslinked organopolysiloxane polymer(s) having no hydrophilic group. The crosslinked organopolysiloxane polymer can be obtained by reaction of alkylhydrogenpolysiloxane and a crosslinking agent that has a reactive vinyl type unsaturated group(s) at the terminal of the molecular chain. Examples of the alkylhydrogenpolysiloxane include linear or partially branched methylhydrogenpolysiloxane, methylhydrogenpolysiloxane in which an alkyl chain with 6 to 20 carbon atoms is grafted, etc. Each of these molecules has to contain two or more hydrogen atoms that are bonded to silicon atom(s) in average. The crosslinking agent is exemplified by a molecule having two or more vinyl type reaction moieties, such as methylvinylpolysiloxane and α,ω-alkenyldiene. Examples thereof include compositions described in each of the following: JP H6-55897B, JP H6-60286B, WO 03/24413A1, and JP 2009-185296A. This crosslinked methylpolysiloxane is swollen with larger weight of oil, for example, low viscosity silicone with the viscosity of 0.65 mm²/second (at 25°C) to 100.0 mm²/second (at 25°C), hydrocarbon oil such as liquid paraffin, squalane, and isododecane, glyceride oil such as trioctanoin, as well as ester oil. Examples of the commercial products of these crosslinked organopolysiloxane include KSG-15, KSG-16, KSG-18, KSG-1610, and USG-103, which are pastes mixed with silicone oil; USG-106, KSG-41, KSG-42, KSG-43, KSG-44, and KSG-810, which are pastes mixed with hydrocarbon oil or triglyceride oil (all of which are manufactured by Shin-Etsu Chemical Co., Ltd.), but not particularly limited thereto. The composition composed of liquid oil material and crosslinked organopolysiloxane having no hydrophilic group is preferably blended in an amount of 0.1 to 50 mass%, more preferably 1 to 30 mass% relative to the total amount of the cosmetic.

The inventive cosmetic may further contain a composition composed of liquid oil material and one or two or more kinds of crosslinked organopolysiloxane polymer having a hydrophilic group. The hydrophilic group is preferably a polyether group or a polyglycerin group. The crosslinked organopolysiloxane polymer having a polyether group and/or a polyglycerin group can be obtained by reaction of alkylhydrogenpolysiloxane and a crosslinking agent that has a reactive vinyl type unsaturated group(s) at the terminal of the molecular chain. Examples of the alkylhydrogenpolysiloxane include methylhydrogenpolysiloxane in which a polyoxyethylene chain is grafted, methylhydrogenpolysiloxane in which a polyglycerin chain is grafted, etc., and the molecule has to contain two or more hydrogen atoms that are bonded to silicon atom(s) in average. This crosslinked organopolysiloxane polymer is swollen with larger weight of low viscosity silicone with the viscosity of 0.65 mm²/second (at 25°C) to 100.0 mm²/second (at 25°C), hydrocarbon oil such as liquid paraffin, squalane, and isododecane, glyceride oil such as trioctanoin, and/or ester oil. The crosslinking agent is exemplified by a molecule having two or more vinyl type reaction moieties such as methylvinylpolysiloxane, α,ω-alkenyldiene, glycerin triallyl ether, polyoxyalkynylated glycerin triallyl ether, trimethylolpropane triallyl ether, and polyoxyalkynylated trimethylolpropane triallyl ether, provided that the crosslinked product by the reaction thereof contains at least one hydrophilic group. Preferable examples of the composition include those described in JP 2631772B, JP H9-136813A, JP 2001-342255A, WO 03/20828A1, and JP 2009-185296A. Examples of the commercial products of these crosslinked organopolysiloxane include KSG-210, KSG-240, and KSG-710, which are pastes mixed with silicone oil; KSG-310, KSG-320, KSG-330, KSG-340, KSG-820, KSG-830, and KSG-840, which are pastes mixed with hydrocarbon oil or triglyceride oil (all of which are manufactured by Shin-Etsu Chemical Co., Ltd.), but not particularly limited thereto. The composition composed of liquid oil and crosslinked organopolysiloxane having a hydrophilic group(s) is preferably blended in an amount of 0.1 to 50 mass%, more preferably 1 to 30 mass% relative to the total amount of the cosmetic.

The inventive cosmetic may contain silicone wax in accordance with the object thereof. This silicone wax is preferably polylactone-modified polysiloxane having bonded polylactone, which is a ring opening polymerization product of a lactone compound having a ring of five or more atoms. Alternatively, this silicone wax is preferably acrylic-modified polysiloxane with the molecule containing at least one functional group selected from a pyrrolidone group, a long-chain alkyl group, a polyoxyalkylene group, a fluoroalkyl group, and anionic groups such as a carboxylic acid. Examples of commercial products thereof include KP-561P and KP-562P (all of which are manufactured by Shin-Etsu Chemical Co., Ltd.) as wax having a long-chain alkyl group.

The inventive cosmetic can also contain an oil-soluble gelation agent in accordance with the object thereof. Examples of the oil-soluble gelation agent include one or two or more kinds of oil-soluble gelation agent(s) selected from metal soap such as aluminum stearate, magnesium stearate, and zinc myristate; an amino acid derivative such as N-lauroyl-L-glutamic acid and α,γ-di-n-butyl amine; dextrin fatty acid ester such as dextrin palmitate, dextrin stearate, and dextrin 2-ethylhexanoate palmitate; sucrose fatty acid ester such as sucrose palmitate and sucrose stearate; fructo-oligosaccharide fatty acid ester such as fructo-oligosaccharide stearate and fructo-oligosaccharide 2-ethylhexanoate; a benzylidene derivative of sorbitol such as monobenzylidene sorbitol and dibenzylidene sorbitol; and an organic-modified clay mineral such as dimethyl benzyl dodecyl ammonium montmorillonite clay and dimethyl dioctadecyl ammonium montmorillonite clay.

The inventive cosmetic includes cosmetics in which the cosmetic component(s) described above are blended, such as make-up cosmetics including make-up foundation, concealer, white powder, liquid foundation, oil foundation, rouge, eye shadow, mascara, eye liner, eyebrow pencil, and a lipstick; hair cosmetics including shampoo, rinse, treatment, and setting material; UV-protective cosmetics including antiperspirant, sunscreen oil, sunscreen lotion, and sunscreen cream.

These cosmetics may be in various forms, such as liquid, emulsion, cream, solid, paste, gel, powder, pressed, laminated, mousse, spray, and stick forms.

Furthermore, these cosmetics may be in various types, such as a water-base, an oil-base, a water-in-oil emulsion, an oil-in-water emulsion, a non-aqueous emulsion, a multi-emulsion including W/O/W and O/W/O.

### EXAMPLE

Hereinafter, the present invention will be described specifically with reference to Synthesis Examples, together with Examples of the inventive cosmetic and Comparative Examples, but the present invention is not limited to the following Examples. Incidentally, "%" described below means "mass%" and represents the content of each component in mass% letting the total mass in each Example be 100% unless otherwise specifically noted. The viscosity is a value at 25°C measured by a Cannon-Fenske-type viscometer.

### Synthetic Example (1)

Into a glass flask equipped with a stirrer, a thermometer, and a reflux condenser, 150 g of 2-allylphenol and 0.04 g of a 3% 2-propanol solution of a chloroplatinic acid-tetramethyldivinyldisiloxane complex were introduced and the temperature was raised to 80°C. While stirring, 248 g of heptamethyl hydrogen trisiloxane was dropped in, and matured at 100°C for 3 hours. The obtained reactant was heated under reduced pressure at 150°C by nitrogen bubbling to remove unreacted raw material, and an organopolysiloxane having the following structure as a light brown transparent liquid with a viscosity of 24 mm²/s were obtained.

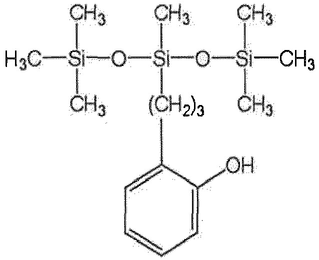

### Synthetic Example (2)

Into a glass flask equipped with a stirrer, a thermometer, and a reflux condenser, 53 g of eugenol and 0.01 g of a 3% 2-propanol solution of a chloroplatinic acid-tetramethyldivinyldisiloxane complex were introduced and the temperature was raised to 80°C. While stirring, 73 g of heptamethyl hydrogen trisiloxane was dropped in, and matured at 100°C for 3 hours. The obtained reactant was heated under reduced pressure at 160°C by nitrogen bubbling to remove unreacted raw material, and an organopolysiloxane having the following structure as a light brown transparent liquid with a viscosity of 19 mm²/s were obtained.

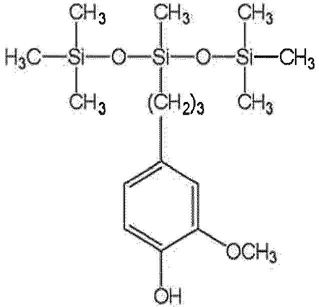

### Synthetic Example (3)

Into a glass flask equipped with a stirrer, a thermometer, and a reflux condenser, 78 g of 2-allylphenol and 0.01 g of a 3% 2-propanol solution of a chloroplatinic acid-tetramethyldivinyldisiloxane complex were introduced and the temperature was raised to 80°C. While stirring, 40 g of tetramethyl dihydrogen disiloxane was dropped in, and matured at 100°C for 3 hours. The obtained reactant was heated under reduced pressure at 150°C by nitrogen bubbling to remove unreacted raw material, and an organopolysiloxane having the following structure as a light brown transparent liquid with a viscosity of 850 mm²/s were obtained.

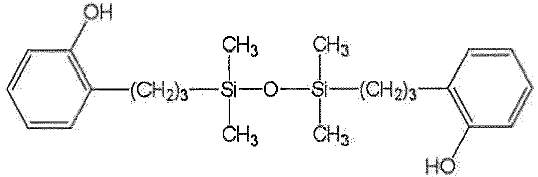

### Synthetic Example (4)

Into a glass flask equipped with a stirrer, a thermometer, and a reflux condenser, 295 g of 2-allylphenol and 0.05 g of a 3% 2-propanol solution of a chloroplatinic acid-tetramethyldivinyldisiloxane complex were introduced and the temperature was raised to 80°C. While stirring, 356 g of dimethylsiloxane pentamer having SiH on both terminals was dropped in, and matured at 100°C for 3 hours. The obtained reactant was heated under reduced pressure at 150°C by nitrogen bubbling to remove unreacted raw material, and an organopolysiloxane having the following structure as a light brown transparent liquid with a viscosity of 410 mm²/s were obtained.

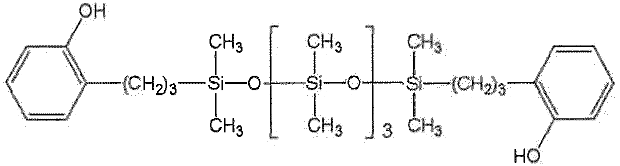

### Comparative Synthetic Example (1)

Into a glass flask equipped with a stirrer, a thermometer, and a reflux condenser, 130 g of α-methylstyrene and 0.02 g of a 3% 2-propanol solution of a chloroplatinic acid-tetramethyldivinyldisiloxane complex were introduced and the temperature was raised to 80°C. While stirring, 222 g of heptamethyl hydrogen trisiloxane was dropped in, and matured at 100°C for 3 hours. The obtained reactant was heated under reduced pressure at 140°C by nitrogen bubbling to remove unreacted raw material, and an organopolysiloxane having the following structure was obtained.

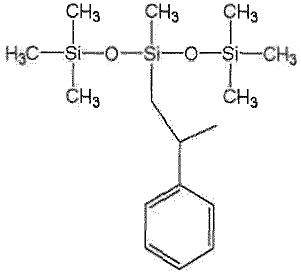

### Comparative Synthetic Example (2)

Into a glass flask equipped with a stirrer, a thermometer, and a reflux condenser, 300 g of 2-allylphenol and 0.06 g of a 3% 2-propanol solution of a chloroplatinic acid-tetramethyldivinyldisiloxane complex were introduced and the temperature was raised to 80°C. While stirring, 504 g of dimethylsiloxane heptamer having SiH on both terminals was dropped in, and matured at 100°C for 3 hours. The obtained reactant was heated under reduced pressure at 150°C by nitrogen bubbling to remove unreacted raw material, and an organopolysiloxane having the following structure was obtained.

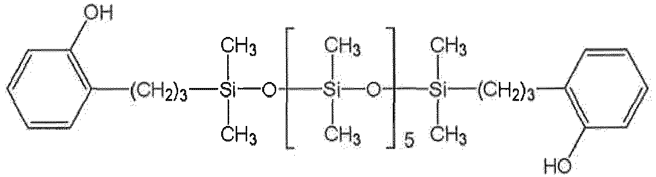

### (Examples 1 to 6 and Comparative Examples 1 to 6)

### [Solubility test]

Each of the components in Tables 1 and 2 was introduced into a 25 ml glass bottle, heated to 80°C, and stirred to prepare a cosmetic. Next, this was cooled to room temperature, left for a week, and observed for the presence or absence of precipitation of the components. The results are shown in Tables 1 and 2. Precipitation state:
Good: precipitation of the components was not observed.
Bad: precipitation of the components was observed.

**[Table 1]**

| | Example 1 | Example 2 | Example 3 | Example 4 | Comparative Example 1 | Comparative Example 2 | Comparative Example 3 | Comparative Example 4 |
|---|---|---|---|---|---|---|---|---|
| Organopolysiloxane of Synthetic Example 1 | 8 | | | | | | | |
| Organopolysiloxane of Synthetic Example 2 | | 8 | | | | | | |
| Organopolysiloxane of Synthetic Example 3 | | | 6 | | | | | |
| Organopolysiloxane of Synthetic Example 4 | | | | 7 | | | | |
| Organopolysiloxane of Comparative Synthetic Example 1 | | | | | 8 | | | |
| Organopolysiloxane of Comparative Synthetic Example 2 | | | | | | 7 | | |
| Diphenylsiloxy phenyl trimethicone* | | | | | | | 8 | |
| Octyl methoxycinnamate | | | | | | | | 6 |
| 4-tert-butyl-4'-methoxydibenzoylmethane | 2 | 2 | 4 | 3 | 2 | 3 | 2 | 4 |
| Total | 10 | 10 | 10 | 10 | 10 | 10 | 10 | 10 |
| Precipitation state | Good | Good | Good | Good | Bad | Bad | Bad | Bad |

Diphenylsiloxy phenyl trimethicone: KF-56A (manufactured by Shin-Etsu Chemical Co., Ltd.)

**[Table 2]**

| | Example 5 | Example 6 | Comparative Example 5 | Comparative Example 6 |
|---|---|---|---|---|
| Organopolysiloxane of Synthetic Example 1 | 5 | | | |
| Organopolysiloxane of Synthetic Example 3 | | 2 | | |
| Organopolysiloxane of Comparative Synthetic Example 1 | | | 5 | |
| Organopolysiloxane of Comparative Synthetic Example 2 | | | | 5 |
| 2,4-bis-{[4-(2-ethyl-hexyloxy)-2-hydroxy]-phenyl}-6-(4-methoxyphenyl)-1,3,5-triazine | 5 | 2 | 5 | 5 |
| Decamethyl cyclopentasiloxane | 10 | 10 | 10 | 10 |
| Total | 20 | 14 | 20 | 20 |
| Precipitation state | Good | Good | Bad | Bad |

It was confirmed that cosmetics that contain the organopolysiloxane (A) of the present invention have high stability.

### (Examples 7 to 10 and Comparative Examples 7 to 9)

Using the components shown in the following Table 3, a sunscreen cosmetic was prepared in accordance with the following production method.

### (Production method)

Step A: Components 1 to 12 were mixed with heating.
Step B: Components 13 to 16 were mixed homogeneously.
Step C: B was added to A while stirring.

### [Method for sensory evaluation]

Each sunscreen cosmetic prepared was applied to skin sufficiently, dried, and then evaluated. This was subjected to sensory evaluation of stability after one month at room temperature, absence of stickiness, smooth spreadability, absence of roughness, cosmetic sustainability, and adherence. These results were represented by the following criteria based on the number of panelists who answered that "it was effective".

### [Evaluation criteria]

Exc. (excellent): 4 to 5 people answered that "it was effective"
Good: 3 people answered that "it was effective"
Fair: 2 people answered that "it was effective"
Bad: 0 or 1 people answered that "it was effective"

**[Table 3]**

| | Component | Examples | | | | Comparative Examples | | |
|---|---|---|---|---|---|---|---|---|
| | | 7 | 8 | 9 | 10 | 7 | 8 | 9 |
| 1 | Organopolysiloxane of Synthetic Example (1) | 15 | | | | | | |
| 2 | Organopolysiloxane of Synthetic Example (2) | | 12 | | | | | |
| 3 | Organopolysiloxane of Synthetic Example (3) | | | 10 | | | | |
| 4 | Organopolysiloxane of Synthetic Example (4) | | | | 5 | | | |
| 5 | Organopolysiloxane of Comparative Synthetic Example (1) | | | | | 15 | | |
| 6 | Organopolysiloxane of Comparative Synthetic Example (2) | | | | | | 5 | |
| 7 | Decamethyl cyclopentasiloxane | 20 | 25 | 20 | 15 | 20 | 15 | 30 |
| 8 | Octyl methoxycinnamate | 3 | 7 | 7 | 6 | 3 | 6 | 7 |
| 9 | 2,4-bis-{[4-(2-ethyl-hexyloxy)-2-hydroxy]-phenyl}-6-(4-methoxyphenyl )-1,3,5-triazine | 4 | 3.5 | 3.5 | 3.5 | 4 | 3.5 | 3.5 |
| 10 | Alkyl-polyether-modified silicone* | 2 | 2 | 2 | 2 | 2 | 2 | 2 |
| 11 | Isododecane | | | | 5 | | 5 | |
| 12 | Isotridecyl isononanoate | 2 | | | 2 | 2 | 2 | |
| 13 | 1,3-butanediol | 10 | 10 | 10 | 10 | 10 | 10 | 10 |
| 14 | Concentrated glycerin | 5 | 5 | 5 | 5 | 5 | 5 | 5 |
| 15 | Disodium edetate hydrate | 0.1 | 0.1 | 0.1 | 0.1 | 0.1 | 0.1 | 0.1 |
| 16 | Purified water | balance | balance | balance | balance | balance | balance | balance |
| | Total | 100 | 100 | 100 | 100 | 100 | 100 | 100 |
| Evaluation | Stability | Exc. | Exc. | Exc. | Good | Bad | Fair | Bad |
| | Non-stickiness | Exc. | Good | Exc. | Good | Good | Fair | Fair |
| | Smooth spreadability | Exc. | Good | Exc. | Exc. | Fair | Bad | Bad |
| | Non-roughness | Exc. | Exc. | Good | Good | Bad | Fair | Bad |
| | Cosmetic sustainability | Good | Good | Good | Good | Good | Fair | Fair |
| | Adherence | Exc. | Good | Good | Exc. | Good | Fair | Bad |

Alkyl-polyether-modified silicone: KF-6038 manufactured by Shin-Etsu Chemical Co., Ltd.

As described in the above results, it was confirmed that the sunscreen cosmetics of the present invention have high stability (no precipitation of components), no stickiness, smooth spreadability, no roughness, favorable cosmetic sustainability, and adherence.

### (Example 11) Sun-cut cream

| (Component) | mass (%) |
|---|---|
| 1. Dimethylpolysiloxane 6cs | 17.5 |
| 2. Organopolysiloxane of Synthetic Example (1) | 14.0 |
| 3. 2-[4-(diethylamino)-2-hydroxybenzoyl]benzoic acid hexyl ester | 3.0 |
| 4. Octyl paramethoxycinnamate | 6.0 |
| 5. Crosslinked polyether-modified silicone (*1) | 5.0 |
| 6. Polyether-modified silicone (*2) | 1.0 |
| 7. Oleophilic-treated zinc oxide | 20.0 |
| 8. Sodium chloride | 0.5 |
| 9. 1,3-Butylene glycol | 2.0 |
| 10. Antiseptic | appropriate |
| 11. Fragrance | appropriate |
| 12. Purified water | 31.0 |
| Total | 100.0 |

| | |
|---|---|
| (*1) Crosslinked polyether-modified silicone: KSG-210 (manufactured by Shin-Etsu Chemical Co., Ltd.) (*2) Polyether-modified silicone: KF-6017 (manufactured by Shin-Etsu Chemical Co., Ltd.) | |

### (Production method)

A: Component 2 was added to part of component 1 and homogenized. Component 7 was added thereto and dispersed with a bead mill.
B: The remainder of component 1 and components 3 to 6 were mixed homogeneously.
C: Components 8 to 10 and component 12 were mixed to be dissolved homogeneously with each other.
D: C was added to B to be emulsified, and A and component 11 were added thereto to obtain a sun-cut cream.

It was confirmed that the sun-cut cream obtained as described above was free from stickiness, lightly spreadable, superior in adherence to be set favorably, and particularly excellent in cosmetic sustainability to be very stable with temperature or time.

### (Example 12) Mascara

| (Component) | mass (%) |
|---|---|
| 1. MQ resin (*1) | 6.0 |
| 2. Branched polyether-modified silicone (*2) | 2.0 |
| 3. Organic-modified clay mineral (*3) | 5.0 |
| 4. Carnauba wax | 7.0 |
| 5. Bees wax | 6.0 |
| 6. Isododecane | 51.0 |
| 7. Organopolysiloxane of Synthetic Example (3) | 10.0 |
| 8. 2,4,6-tris[4-(2-ethylhexyloxycarbonyl)anilino]-1,3,5-triazine | 3.0 |
| 9. Silicone-treated black iron oxide (*4) | 5.0 |
| 10. Silicone-treated talc (*4) | 5.0 |
| 11. Antiseptic | appropriate |
| Total | 100.0 |

| | |
|---|---|
| (*1) MQ resin: KF-7312J (manufactured by Shin-Etsu Chemical Co., Ltd.) (*2) Branched polyether-modified silicone: KF-6028P (manufactured by Shin-Etsu Chemical Co., Ltd.) (*3) Organic-modified clay mineral:: BENTON 38VCG (manufactured by Elementis Specialties) (*4) Silicone-treated black iron oxide, talc: treated by KF-9909 (manufactured by Shin-Etsu Chemical Co., Ltd.) | |

### (Production method)

A: Components 1 to 11 were mixed homogeneously while heating and stirring to obtain mascara.

It was confirmed that the mascara obtained as described above had no stickiness, had favorable spreadability, and was particularly excellent in cosmetic sustainability to be very stable with temperature or time.

### (Example 13) Cleansing gel

| (Component) | mass (%) |
|---|---|
| 1. Hydroxypropyl methylcellulose | 5.0 |
| 2. Purified water | balance |
| 3. Polyoxyethylene glyceryl triisostearate (20EO) | 8.0 |
| 4. Polyoxyethylene Lauryl Ether (5EO) | 5.0 |
| 5. Organopolysiloxane of Synthetic Example (1) | 5.0 |
| 6. 1,3-butylene glycol | 10.0 |
| 7. Ethanol | 5.0 |
| 8. Methylparaben | 0.1 |
| 9. Fragrance | appropriate |
| Total | 100.0 |

### (Production method)

Components 1 to 9 were mixed homogeneously at a normal temperature to obtain a cleansing gel.

The cleansing gel of Example 13 had a refreshing feeling, a smooth feeling of use, and was excellent in washing by water.

### (Example 14) Stick-type lipstick

| (Component) | mass (%) |
|---|---|
| 1. Polyethylene wax | 7.0 |
| 2. Microcrystalline wax | 3.0 |
| 3. Ceresin was | 2.0 |
| 4. Glyceryl tri-2 ethylhexanoate | 20.0 |
| 5. Pentaerythrit tetra 2-ethylhexanoate | 10.0 |
| 6. Dimethylpolysiloxane | 3.0 |
| 7. Cetyl 2-ethylhexanoate | balance |
| 8. Organopolysiloxane of Synthetic Example (1) | 3.0 |
| 9. Red No. 202 | 0.5 |
| 10. Yellow No. 4 | 2.0 |
| 11. Titanium oxide | 0.5 |
| 12. Black iron oxide | 0.1 |
| 13. Phenoxyethanol | 0.2 |
| 14. Fragrance | appropriate |
| Total | 100.0 |

### (Production method)

(1) Components 1 to 8 were dissolved and mixed homogeneously at 100°C.
(2) Components 9 to 14 were added to (1) and mixed homogeneously.
(3) (2) was poured into a container and cooled to obtain a stick-type lipstick.

The stick-type lipstick of Example 14 had a smooth feeling of use, adhesion, and excellent cosmetic sustainability.

It should be noted that the present invention is not limited to the above-described embodiments. The embodiments are just examples, and any examples that have substantially the same feature and demonstrate the same functions and effects as those in the technical concept disclosed in claims of the present invention are included in the technical scope of the present invention.

## Claims

1. A cosmetic comprising:
(A) an organo(poly)siloxane shown by the following general formula (1): 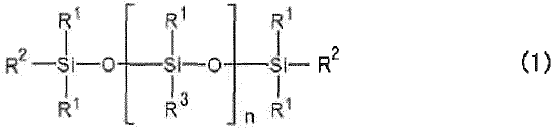 wherein, in the formula (1), R¹ independently represents a group except shown by the following general formula (2), selected from a group consisting of a substituted or unsubstituted alkyl group having 1 to 20 carbon atoms and a substituted or unsubstituted aryl group having 6 to 20 carbon atoms; n represents an integer of 0 to 3; R² and R³ each independently represent R¹ or an organic group shown by the following general formula (2), provided that either or both of R² and R³ in the formula (1) represent the organic group shown by the following general formula (2), 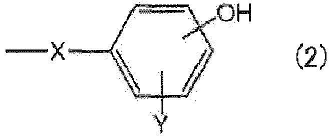 wherein, in the formula (2), X represents a substituted or unsubstituted alkylene group having 2 to 20 carbon atoms; Y represents any one selected from a group consisting of a hydrogen atom, a substituted or unsubstituted, linear, branched, or cyclic alkyl group having 1 to 20 carbon atoms, a substituted or unsubstituted aryl group having 6 to 20 carbon atoms, a substituted or unsubstituted aralkyl group having 7 to 40 carbon atoms, a substituted or unsubstituted alkyloxy group having 1 to 20 carbon atoms, a substituted or unsubstituted aryloxy group having 6 to 20 carbon atoms, a substituted or unsubstituted arylalkyloxy group having 7 to 40 carbon atoms, and a halogen atom; and an OH group in a phenyl group in the formula (2) substitutes independently at an ortho, meta, or para position.

2. The cosmetic according to claim 1, further comprising (B) an organic UV-absorber.

3. The cosmetic according to claim 2, wherein (B) the organic UV-absorber is one or more kinds selected from a group consisting of a triazine derivative, a benzoic ester derivative, a benzophenone derivative, and a dibenzoylmethane derivative.

4. The cosmetic according to any one of claims 1 to 3, further comprising (C) a silicone oil other than (A) the organo(poly)siloxane.

5. The cosmetic according to any one of claims 1 to 4, wherein a content of (A) the organo(poly)siloxane is 0.1 to 50 mass%.

6. The cosmetic according to any one of claims 1 to 5, further comprising (D) an ester oil.
